(19) Europäisches Patentamt
European Patent Office
Office européen des brevets

(11) EP 0 877 726 B1

(12) FASCICULE DE BREVET EUROPEEN

(45) Date de publication et mention de la délivrance du brevet:
**12.12.2001 Bulletin 2001/50**

(21) Numéro de dépôt: **96932642.0**

(22) Date de dépôt: **24.09.1996**

(51) Int Cl.[7]: **C07C 45/46**, C07C 309/06

(86) Numéro de dépôt international:
**PCT/FR96/01488**

(87) Numéro de publication internationale:
**WO 97/11930 (03.04.1997 Gazette 1997/15)**

(54) **PROCEDE D'ACYLATION D'UN COMPOSE AROMATIQUE**

VERFAHREN ZUR ACYLIERUNG EINER AROMATISCHEN VERBINDUNG

AROMATIC COMPOUND ACYLATION METHOD

(84) Etats contractants désignés:
**AT BE CH DE DK ES FI FR GB IE IT LI NL PT**

(30) Priorité: **25.09.1995 FR 9511250**

(43) Date de publication de la demande:
**18.11.1998 Bulletin 1998/47**

(73) Titulaire: **RHODIA CHIMIE**
**92408 Courbevoie Cédex (FR)**

(72) Inventeurs:
- **DUBAC, Jacques**
  **F-31320 Pechbusque (FR)**
- **GASPARD, Hafida**
  **F-31400 Toulouse (FR)**
- **LABROUILLERE, Mireille**
  **F-47000 Agen (FR)**
- **LAPORTERIE, André**
  **F-31450 Pompertuzat (FR)**
- **DESMURS, Jean-Roger**
  **F-69360 Saint-Symphorien-d'Ozon (FR)**
- **LE ROUX, Christophe**
  **F-31500 Toulouse (FR)**

(74) Mandataire: **Dubruc, Philippe**
**Rhodia Services,**
**Direction de la Propriété Industrielle,**
**40, reu de la Haie-Coq**
**93306 Aubervilliers Cedex (FR)**

(56) Documents cités:
**EP-A- 0 067 698**
- **J. CHEM. SOC., CHEM. COMMUN. (JCCCAT,00224936);93; (14); PP.1157-8, NIPPON STEEL CORP.;ADV. MATER. LAB.; KAWASAKI; 211; JAPAN (JP), XP000564618 KAWADA A ET AL: "Lanthanide trifluoromethanesulfonates as reusable catalysts: catalytic Friedel-Crafts acylation" cité dans la demande**
- **JOURNAL OF THE CHEMICAL SOCIETY, PERKIN TRANSACTIONS 1, no. 20, 1995, LETCHWORTH GB, pages 2543-2549, XP002020312 Y. MATANO ET AL.: "Synthesis, x-ray structure, thermal stability and reactions of triaryl(3-oxoalkyl)bismuthonium salts"**
- **INDIAN J. CHEM., SECT. A (IJCADU,03764710);83; VOL.22A (9); PP.814-15, PANJAB UNIV.;DEP. CHEM.; CHANDIGARH; 160 014; INDIA (IN), XP000572904 SINGH S ET AL: "Trifluoromethanesulfonates of antimony(III) and bismuth(III)"**
- **J. FLUORINE CHEM. (JFLCAR,00221139);90; VOL.48 (3); PP.421-8, PANJAB UNIV.;DEP. CHEM.; CHANDIGARH; 160 104; INDIA (IN), XP002005239 NIYOGI D G ET AL: "Some reactions of trifluoromethanesulfonic anhydride with various metal substrates"**
- **JOURNAL OF FLUORINE CHEMISTRY, vol. 70, no. 2, 1995, LAUSANNE CH, pages 237-240, XP002020313 D.G. DEBYANI ET AL.: "Reactions of fluorinated acid anhydrides, (CF3CO)2O, (CF3SO2)2O and (FSO2)2O, with organometallic substrates of group 15 (As, Sb and Bi)"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

(Cont. page suivante)

Remarques:

Le dossier contient des informations techniques présentées postérieurement au dépôt de la demande et ne figurant pas dans le présent fascicule.

## Description

La présente invention a pour objet un procédé d'acylation d'un composé aromatique. Plus précisément, l'invention a trait à un procédé d'acylation d'un composé aromatique activé ou désactivé.

Elle est particulièrement intéressante dans le cas où il est souhaité d'acyler un composé aromatique désactivé.

L'invention s'applique à la préparation de cétones aromatiques.

L'invention vise également la préparation du catalyseur et de nouveaux composés du bismuth.

Dans l'exposé qui suit de la présente invention, on entend "par composé aromatique", la notion classique d'aromaticité telle que définie dans la littérature, notamment par Jerry MARCH, Advanced Organic Chemistry, 4$^{ème}$ édition, John Wiley and Sons, 1992, pp. 40 et suivantes.

Par "composé aromatique désactivé", on définit un composé aromatique sans substituant, tel que par exemple le benzène ou un composé aromatique comportant un ou plusieurs substituants désactivant le noyau aromatique tels que des groupes électro-attracteurs.

Par "composé aromatique activé", on désigne un composé aromatique comportant un ou plusieurs substituants activant le noyau aromatique tels que des groupes électro-donneurs.

Les notions de groupes électro-attracteurs et groupes électro-donneurs sont définies dans la littérature. On peut se référer entre autres, à l'ouvrage de Jerry MARCH - Advanced Organic Chemistry, 4$^{ème}$ édition, John Wiley and Sons, 1992, chapitre 9, pp. 273 - 292.

Un procédé classique de préparation de cétones aromatiques consiste à effectuer une réaction d'acylation de type Friedel-Crafts.

On fait réagir le composé aromatique et un agent d'acylation, en présence d'un catalyseur qui est généralement le chlorure d'aluminium.

Une illustration de ce type de procédé est donné par les travaux de C. KURODA et al [Sci. Papers Inst. Phys. Chem. Res. 18, pp. 51-60 (1932)] qui ont décrit la préparation de méthoxyacétophénones, par réaction d'un composé aromatique porteur de 1 à 3 groupes méthoxy, avec du chlorure d'acétyle, en présence de chlorure d'aluminium.

Toutefois, la mise en oeuvre du chlorure d'aluminium présente de nombreux inconvénients. Le chlorure d'aluminium est un produit corrosif et irritant. De plus, il est nécessaire de mettre en oeuvre une quantité importante de chlorure d'aluminium au moins égale à la stoechiométrie, par suite de la complexation de la cétone formée. En conséquence, le chlorure d'aluminium n'est donc pas un vrai catalyseur.

En fin de réaction, il est nécessaire d'éliminer le chlorure d'aluminium du milieu réactionnel en faisant une hydrolyse acide ou basique.

Cette technique d'hydrolyse implique l'addition d'eau dans le milieu réactionnel ce qui complique notablement la mise en oeuvre du procédé car le cation métallique, et plus particulièrement le cation aluminium forme alors en présence d'eau, des complexes polyoxo- et/ou polyhydroxo- d'aluminium de consistance laiteuse, difficiles ensuite à séparer. Il en résulte la nécessité de faire un traitement long et coûteux comportant après l'hydrolyse, une extraction de la phase organique, une séparation des phases aqueuse et organique, voire-même un séchage de cette dernière. La séparation du chlorure d'aluminium est donc longue et coûteuse.

Par ailleurs, se pose le problème des effluents aqueux salins qu'il faut ensuite neutraliser ce qui impose une opération supplémentaire.

De plus, le chlorure d'aluminium ne peut être recyclé du fait de son hydrolyse.

Pour pallier cet inconvénient, Atsushi KAWADA et al [J. Chem. Soc. Chem. Commun. pp. 1157-1158 (1993) et Synlett pp.545-546 (1994)] ont proposé d'effectuer la réaction d'acylation d'un composé aromatique par l'anhydride acétique en présence d'une quantité catalytique de trifluorométhanesulfonate de lanthanide, en particulier d'ytterbium ou de triflate de scandium.

L'inconvénient dont souffrent ces catalyseurs est qu'ils ne permettent d'acyler que des composés aromatiques activés, tels que l'anisole.

Ces dernières références, comme de nombreux catalyseurs décrits dans l'état de la technique, ne traitent pas d'une façon générale, le problème de l'acylation de substrats aromatiques aussi bien activés que désactivés et dans des conditions aisément mises en oeuvre.

La présente invention atteint cet objectif et fournit un procédé permettant d'obvier aux inconvénients précités.

Il a maintenant été trouvé et c'est ce qui constitue l'objet de la présente invention, un procédé d'acylation d'un composé aromatique qui consiste à faire réagir ledit composé aromatique avec un agent d'acylation, en présence d'un catalyseur caractérisé par le fait que l'on conduit la réaction d'acylation en présence d'une quantité efficace d'au moins un sel de bismuth de l'acide triflique.

Dans le présent texte, on entend par "acide triflique", l'acide trifluorométhanesulfonique $CF_3SO_3H$.

Comme mentionné ci-après, l'invention envisage, à titre de catalyseurs, le tris-trifluorométhanesulfonate de bismuth mais également les catalyseurs comprenant moins de 3 anions trifluorométhanesulfonate pour un cation bismuth.

Un autre objet de l'invention est un procédé de préparation du catalyseur de l'invention.

Enfin, un autre objet de l'invention réside dans de nouveaux composés du bismuth.

Plus précisément, la présente invention a pour objet un procédé d'acylation d'un composé aromatique répondant à la formule générale (I):

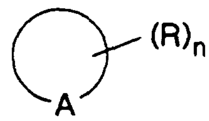

(I)

dans laquelle :

- A symbolise le reste d'un cycle formant tout ou partie d'un système carbocyclique ou hétérocyclique, aromatique, monocyclique ou polycyclique : ledit reste cyclique pouvant porter un radical R représentant un atome d'hydrogène ou un ou plusieurs substituants, identiques ou différents,
- n représente le nombre de substituants sur le cycle.

L'invention s'applique notamment aux composés aromatiques répondant à la formule (I) dans laquelle A est le reste d'un composé cyclique, ayant de préférence, au moins 4 atomes dans le cycle, éventuellement substitué, et représentant au moins l'un des cycles suivants :

. un carbocycle aromatique, monocyclique ou polycyclique,
. un hétérocycle aromatique, monocyclique ou polycyclique comportant au moins un des hétéroatomes O, N et S,

On précisera, sans pour autant limiter la portée de l'invention, que le reste A éventuellement substitué représente, le reste :

1° - d'un composé carbocyclique aromatique, monocyclique ou polycyclique.

Par "composé carbocyclique polycyclique", on entend :

. un composé constitué par au moins 2 carbocycles aromatiques et formant entre eux des systèmes ortho- ou ortho- et péricondensés,
. un composé constitué par au moins 2 carbocycles dont l'un seul d'entre eux est aromatique et formant entre eux des systèmes ortho- ou ortho- et péricondensés.

2° - d'un composé hétérocyclique aromatique, monocyclique ou polycyclique.

Par "composé hétérocyclique polycyclique", on définit :

. un composé constitué par au moins 2 hétérocycles contenant au moins un hétéroatome dans chaque cycle dont au moins l'un des deux cycles est aromatique et formant entre eux des systèmes ortho- ou ortho- et péricondensés,
. un composé constitué par au moins un cycle hydrocarboné et au moins un hétérocycle dont au moins l'un des cycles est aromatique et formant entre eux des systèmes ortho- ou ortho- et péricondensés.

3° - d'un composé constitué par un enchaînement de cycles, tels que définis aux paragraphes 1 et/ou 2 liés entre eux :

. par un lien valentiel,
. par un radical alkylène ou alkylidène ayant de 1 à 4 atomes de carbone, de préférence un radical méthylène ou isopropylidène,
. par l'un des groupes suivants :

-O-, -CO-, -COO-, -OCOO-

-S-, -SO-, $-SO_2-$,

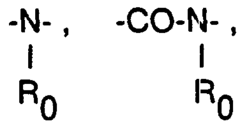

dans ces formules, $R_0$ représente un atome d'hydrogène ou un radical alkyle ayant de 1 à 4 atomes de carbone, un radical cyclohexyle ou phényle.

Comme exemples de cycles sous 1° à 3°, on peut citer :

1° - le benzène, toluène, xylène, naphtalène, anthracène,
2° - le furane, pyrrole, thiofène, isoxazole, furazanne, isothiazole, imidazole, pyrazole, pyridine, pyridazine, pyrimidine, quinoléïne, naphtyridine, benzofurane, indole,
3°-le biphényle, 1,1'-méthylènebiphényle, 1,1'-isopropylidènebiphényle, 1,1'-oxybiphényle, 1,1'-iminobiphényle.

Dans le procédé de l'invention, on met en oeuvre préférentiellement un composé aromatique de formule (I) dans laquelle A représente un noyau benzénique.
Le composé aromatique de formule (I) peut être porteur d'un ou plusieurs substituants.
Le nombre de substituants présents sur le cycle dépend de la condensation en carbone du cycle et de la présence ou non d'insaturations sur le cycle.
Le nombre maximum de substituants susceptibles d'être portés par un cycle, est aisément déterminé par l'Homme du Métier.
Dans le présent texte, on entend par "plusieurs", généralement, moins de 4 substituants sur un noyau aromatique. Des exemples de substituants sont donnés ci-dessous mais cette liste ne présente pas de caractère limitatif. Comme mentionné précédemment, les substituants peuvent activer ou non le noyau aromatique.
Le reste A peut être éventuellement porteur d'un ou plusieurs substituants qui sont représentés dans la formule (I), par le symbole R et dont les significations préférées sont définies ci-après :

- le ou les radicaux R représentent l'un des groupes suivants :

  . un atome d'hydrogène,
  . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  . un radical cyclohexyle,
  . un groupe acyle ayant de 2 à 6 atomes de carbone,
  . un radical de formule :

$$-R_1-OH$$

$$-R_1-COOR_2$$

$$-R_1-CHO$$

$$-R_1-NO_2$$

$$-R_1-CN$$

$-R_1-N(R_2)_2$

$-R_1-CO-N(R_2)_2$

$-R_1-X$

$-R_1-CF_3$

dans lesdites formules, $R_1$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; les radicaux $R_2$ identiques ou différents représentent un atome d'hydrogène ou un radical alkyle linéaire ou ramifié ayant de 1 à 6 atomes de carbone ; X symbolise un atome d'halogène, de préférence un atome de chlore, de brome ou de fluor.

Lorsque n est supérieur ou égal à 2, deux radicaux R et les 2 atomes successifs du cycle aromatique peuvent être liés entre eux par un radical alkylène, alcénylène ou alcénylidène ayant de 2 à 4 atomes de carbone pour former un hétérocycle saturé, insaturé ou aromatique ayant de 5 à 7 atomes de carbone. Un ou plusieurs atomes de carbone peuvent être remplacés par un autre hétéroatome, de préférence l'oxygène. Ainsi, les radicaux R peuvent représenter un radical méthylène dioxy ou éthylène dioxy.

La présente invention s'applique tout particulièrement aux composés aromatiques répondant à la formule (I) dans laquelle :

- le ou les radicaux R représentent l'un des groupes suivants :
  - . un atome d'hydrogène
  - . un groupe OH,
  - . un radical alkyle linéaire ou ramifié ayant de 1 à 6 atomes de carbone,
  - . un radical alcényle linéaire ou ramifié ayant de 2 à 6 atomes de carbone,
  - . un radical alkoxy linéaire ou ramifié ayant de 1 à 6 atomes de carbone,
  - . un groupe -CHO,
  - . un groupe acyle ayant de 2 à 6 atomes de carbone,
  - . un groupe $-COOR_2$ où $R_2$ a la signification donnée précédemment,
  - . un groupe $-NO_2$,
  - . un groupe $-NH_2$,
  - . un atome d'halogène, de préférence, de fluor, de chlore, de brome,
  - . un groupe $-CF_3$.
- n est un nombre égal à 0, 1, 2 ou 3.

Parmi les composés de formule (I), on met en oeuvre plus particulièrement ceux répondant aux formules suivantes :

- un composé carbocyclique aromatique monocyclique ou polycyclique avec des cycles pouvant former entre eux un système orthocondensé répondant à la formule (Ia) :

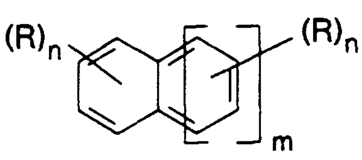

(Ia)

dans ladite formule (Ia), m représente un nombre égal à 0, 1 ou 2 et les symboles R, identiques ou différents et n, ayant la signification donnée précédemment,

- un composé constitué par un enchaînement de deux ou plusieurs carbocycles aromatiques monocycliques répondant à la formule (Ib) :

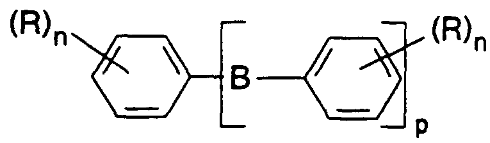

(Ib)

dans ladite formule (Ib), les symboles R, identiques ou différents et n, ont la signification donnée précédemment, p est un nombre égal à 0, 1, 2 ou 3 et B représente :

- un lien valentiel
- un radical alkylène ou alkylidène ayant de 1 à 4 atomes de carbone, de préférence, un radical méthylène ou isopropylidène,
- l'un des groupes suivants :

-O-, -CO- , -COO- , -OCOO-

-S-, -SO-, $-SO_2-$,

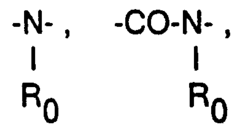

dans ces formules, $R_0$ représente un atome d'hydrogène ou un radical alkyle ayant de 1 à 4 atomes de carbone, un radical cyclohexyle ou phényle.

Les composés de formule (I) mis en oeuvre préférentiellement répondent aux formules (Ia) et (Ib) dans lesquelles :

- R représente un atome d'hydrogène, un groupe hydroxyle, un groupe —CHO, un groupe $-NO_2$, un groupe $-NH_2$, un radical alkyle ou alkoxy linéaire ou ramifié ayant de 1 à 6 atomes de carbone, de préférence, de 1 à 4 atomes de carbone ou un atome d'halogène,
- B symbolise un lien valentiel, un radical alkylène ou alkylidène ayant de 1 à 4 atomes de carbone ou un atome d'oxygène,
- m est égal à 0 ou 1,
- n est égal à 0, ou 2,
- p est égal à 0 ou 1.

Encore plus préférentiellement, on choisit les composés de formule (I) dans laquelle R représente un atome d'hydrogène, un groupe hydroxyle, un radical méthyle, un radical méthoxy ou un atome d'halogène.

A titre illustratif de composés répondant à la formule (I), on peut mentionner plus particulièrement :

- des composés aromatiques halogénés ou non tels que le benzène, le toluène, le chlorobenzène, les dichlorobenzènes, les trichlorobenzènes, le fluorobenzène, les difluorobenzènes, les chlorofluorobenzènes, les chlorotoluènes, les fluorotoluènes, le bromobenzène, les dibromobenzènes, les bromofluorobenzènes, les bromochlorobenzènes, le trifluorométhylbenzène, le trifluorométhoxybenzène, le trichlorométhylbenzène, le trichlorométhoxybenzène, le trifluorométhylthiobenzène,
- des composés aromatiques aminés ou nitrés tels que l'aniline et le nitrobenzène,
- des composés phénoliques tels que le phénol, l'o-crésol, le gaïacol,

- des monoéthers tels que l'anisole, l'éthoxybenzène (phénétole), le butoxybenzène, l'isobutoxybenzène, le 2-chloroanisole, le 3-chloroanisole, le 2-bromoanisole, le 3-bromoanisole, le 2-méthylanisole, le 3-méthylanisole, le 2-éthylanisole, le 3-éthylanisole, le 2-isopropylanisole, le 3-isopropylanisole, le 2-propylanisole, le 3-propylanisole, le 2-allylanisole, le 2-butylanisole, le 3-butylanisole, le 2-tert-butylanisole, le 3-tert-butylanisole, le 2-benzylanisole, le 2-cyclohexylanisole, le 1-bromo-2-éthoxybenzène, le 1-bromo-3-éthoxybenzène, le 1-chloro-2-éthoxybenzène, le 1-chloro-3-éthoxybenzène, le 1-éthoxy-2-éthylbenzène, le 1-éthoxy-3-éthylbenzène, le 2,3-diméthylanisole, le 2,5-diméthylanisole,
- des diéthers comme le vératrole, le 1,3-diméthoxybenzène, le 1,2-diéthoxybenzène, le 1,3-diéthoxybenzène, le 1,2-dipropoxybenzène, le 1,3-dipropoxybenzène, le 1,2-méthylènedioxybenzène, le 1,2-éthylènedioxybenzène,
- des triéthers comme le 1,2,3-triméthoxybenzène, le 1,3,5-triméthoxybenzène, le 1,3,5-triéthoxybenzène.

Les composés auxquels s'applique de manière plus particulièrement intéressante le procédé selon l'invention sont le benzène, le toluène, le phénol, l'anisole et le vératrole.

Pour ce qui est du réactif d'acylation, il répond plus particulièrement à la formule (II) :

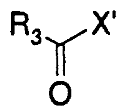
(II)

dans laquelle :

- $R_3$ représente :
  - . un radical aliphatique saturé ou insaturé, linéaire ou ramifié ayant de 1 à 24 atomes de carbone ; un radical cycloaliphatique, saturé, insaturé ou aromatique, monocyclique ou polycyclique, ayant de 4 à 12 atomes de carbone ; un radical aliphatique saturé ou insaturé, linéaire ou ramifié, porteur d'un substituant cyclique,
- X' représente :
  - . un atome d'halogène, de préférence un atome de chlore ou de brome,
  - . un radical $-O-CO-R_4$ avec $R_4$, identique ou différent de $R_3$, ayant la même signification que $R_3$.

Par substituant cyclique, on entend de préférence, un cycle carbocyclique saturé, insaturé ou aromatique, de préférence cycloaliphatique ou aromatique notamment cycloaliphatique comprenant 6 atomes de carbone dans le cycle ou benzénique.

Plus préférentiellement, $R_3$ représente un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome (par exemple, l'oxygène), par un groupe fonctionnel (par exemple —CO—) et/ou porteuse d'un substituant (par exemple, un halogène ou un groupe $CF_3$).

$R_3$ représente de préférence un radical alkyle ayant de 1 à 4 atomes de carbone, tel que méthyle, éthyle, propyle, isopropyle, butyle, isobutyle, sec-butyle, tert-butyle.

Le radical $R_3$ représente également d'une manière préférentielle un radical phényle qui peut être éventuellement substitué. Il est nécessaire que ce radical soit plus désactivé que le composé aromatique car dans le cas contraire, on assisterait à l'acylation de l'agent d'acylation lui-même.

Comme exemples plus particuliers de substituants, on peut citer, notamment :

- . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
- . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,
- . un groupe hydroxyle,
- . un atome d'halogène, de préférence un atome de fluor, chlore ou brome.

Les agents d'acylation préférés répondent à la formule (II) dans laquelle X' représente un atome de chlore et $R_3$ représente un radical méthyle ou éthyle.

Lorsque l'agent d'acylation est un anhydride d'acide, les composés préférés répondent à la formule (II) dans laquelle $R_3$ et $R_4$ sont identiques et représentent un radical alkyle ayant de 1 à 4 atomes de carbone.
A titre illustratif d'agents d'acylation répondant à la formule (II), on peut citer plus particulièrement :

- le chlorure d'acétyle,
- le chlorure de monochloroacétyle,
- le chlorure de dichloroacétyle,
- le chlorure de propanoyle,
- le chlorure d'isobutanoyle,
- le chlorure de pivaloyle,
- le chlorure de stéaroyle,
- le chlorure de crotonyle,
- le chlorure de benzoyle,
- les chlorures de chlorobenzoyle,
- le chlorure de p-nitrobenzoyle
- les chlorures de méthoxybenzoyle,
- les chlorures de naphtoyle,
- l'anhydride acétique,
- l'anhydride isobutyrique,
- l'anhydride trifluoroacétique,
- l'anhydride benzoïque.

Conformément au procédé de l'invention, on effectue la réaction d'acylation d'un composé aromatique en présence d'un catalyseur, un sel de bismuth de l'acide trifluorométhanesulfonique.
On peut mettre en oeuvre ledit sel aussi bien sous forme anhydre que sous forme hydratée.
Le catalyseur mis en oeuvre préférentiellement dans le procédé de l'invention répond à la formule suivante :

$$Ar_n\ Bi(OTf)_{3-n} \qquad (IV)$$

dans ladite formule (IV):

- OTf représente $O_3SCF_3$,
- Ar représente un radical carbocyclique, aromatique, monocyclique ou polyclique,
- n est égal à 0, 1 ou 2.

Ainsi, dans la formule (IV), on entend par "Ar", un radical carbocyclique, aromatique, monocyclique ou polycyclique, éventuellement substitué, ayant de préférence, de 6 à 18 atomes de carbone.
On peut citer plus particulièrement, les radicaux phényle ou naphtyle, éventuellement substitués, par exemple, par des radicaux alkyle ou alkoxy ayant de 1 à 4 atomes de carbone, des atomes d'halogène ou des groupes trifluorométhyle. Comme radicaux Ar, on choisit de préférence, un radical phényle ou tolyle et encore plus préférentiellement un radical phényle.
Les catalyseurs susceptibles d'être mis en oeuvre dans le procédé de l'invention sont aussi bien le tris-trifluorométhylsulfonate de bismuth de formule $Bi(OTf)_3$ que les intermédiaires de fabrication du triflate de bismuth répondant aux formules suivantes : $Bi(OTf)Ar_2$ (IVa) et $Bi(OTf)_2Ar$ (IVb) et plus particulièrement $Bi(OTf)Ph_2$ et $Bi(OTf)_2Ph$.
On peut également mettre en oeuvre un mélange desdits composés.
Comme précisé ci-dessus, le catalyseur peut être $Bi(OTf)_3$ avec OTf représentant $O_3SCF_3$. On l'appelle de manière simplifiée, triflate de bismuth. Ce produit est un produit connu décrit dans la littérature. Par contre, les composés de formule (IV) dans laquelle n = 1 et n = 2, sont nouveaux et revendiqués en tant que tels.
Conformément au procédé d'acylation de l'invention, on peut mettre en oeuvre aussi bien le triflate de bismuth que les nouvelles entités catalytiques répondant à la formule (IVa) et (IVb). Par commodité de langage, on utilisera dans la suite du texte, l'expression "triflate de bismuth", pour désigner aussi bien le tris-trifluorométhanesulfonate de bismuth que les produits intermédiaires de formule (IVa) et (IVb).
On ne sortira pas non plus du cadre de l'invention, à mettre en oeuvre un sel de bismuth d'un degré d'oxydation supérieur, par exemple 5, dans lequel un anion triflate peut être remplacé par un atome d'un métal, de préférence, monovalent et plus particulièrement, le sodium et/ou par un atome d'oxygène. Comme exemples de ce type de catalyseurs, on peut citer entre autres, $NaBi(SO_3CF_3)_4$ et $NaBiO(SO_3CF_3)_2$.

L'invention inclut tout composé du bismuth comprenant un radical de type $R_F$-$SO_3$ - dans lequel $R_F$ représente un radical alkyle polyfluoré ayant au moins 1 atome de carbone jusqu'à un nombre très élevé, ainsi qu'une chaîne alkyle polyfluorée telle que celle d'une résine perfluorée porteuse de groupes sulfoniques.

Conformément au procédé de l'invention, la réaction entre le composé aromatique et l'agent d'acylation, peut être conduite en présence ou en l'absence d'un solvant organique : l'un des réactifs pouvant être utilisé comme solvant réactionnel.

Une variante préférée du procédé de l'invention consiste à conduire la réaction dans un solvant organique.

On choisit de préférence un solvant du substrat de départ et plus préférentiellement, un solvant organique, aprotique, polaire.

Comme exemples de solvants organiques aprotiques, polaires qui peuvent également être mis en oeuvre dans le procédé de l'invention, on peut citer plus particulièrement les carboxamides linéaires ou cycliques comme le N,N-diméthylacétamide (DMAC), le N,N-diéthylacétamide, le diméthylformamide (DMF), le diéthylformannide ou la 1-méthyl-2-pyrrolidinone (NMP) ; les composés nitrés tels que le nitrométhane, le nitroéthane, le 1-nitropropane, le 2-nitropropane ou leurs mélanges, le nitrobenzène ; les nitriles alphatiques ou aromatiques comme l'acétonitrile, le propionitrile, le butanenitrile, l'isobutanenitrile, le benzonitrile, le cyanure de benzyle ; le diméthylsulfoxyde (DMSO) ; la tétraméthylsulfone (sulfolane), la diméthylsulfone, l'hexaméthylphosphotriamide (HMPT) ; la diméthyléthylèneurée, la diméthylpropylèneurée, la tétraméthylurée ; le carbonate de propylène.

Les solvants préférés sont : le nitrométhane, le nitroéthane, le 1-nitropropane, le 2-nitropropane.

On peut également utiliser un mélange de solvants organiques.

Dans une première étape du procédé de l'invention, on effectue l'acylation du composé aromatique. Dans une étape suivante, on conduit l'hydrolyse de la masse réactionnelle obtenue.

Le rapport entre le nombre de moles de composé aromatique et le nombre de moles d'agent d'acylation peut varier car le substrat peut servir de solvant réactionnel. Ainsi, le rapport peut aller de 0,1 à 10, de préférence entre 1,0 et 4,0.

La quantité de catalyseur mis en oeuvre est déterminée de telle sorte que le rapport entre le nombre de moles de catalyseur et le nombre de moles d'agent d'acylation est inférieur à 1,0 et varie, de préférence, entre 0,001 et 0,8, et encore plus préférentiellement entre 0,02 et 0,2.

Pour ce qui est de la quantité de solvant organique mis en oeuvre, elle est choisie généralement de telle sorte que le rapport entre le nombre de moles de solvant organique et le nombre de moles de composé aromatique varie, de préférence, entre 0 et 100 et encore plus préférentiellement entre 0 et 50.

La température à laquelle est mise en oeuvre la réaction d'acylation dépend de la réactivité du substrat de départ et de celle de l'agent d'acylation.

Elle se situe entre 20°C et 200°C, de préférence entre 40°C et 150°C.

Généralement, la réaction est conduite à pression atmosphérique mais des pressions plus faibles ou plus élevées peuvent également convenir.

D'un point de vue pratique, il n'y a pas de contraintes au niveau de la mise en oeuvre des réactifs. Ils peuvent être introduits dans un ordre quelconque.

Après mise en contact des réactifs, on porte le mélange réactionnel à la température souhaitée.

Une autre variante de l'invention consiste à chauffer l'un des réactifs (agent d'acylation ou composé aromatique) avec le catalyseur, puis à introduire l'autre réactif.

La durée de la réaction est fonction de nombreux paramètres. Le plus souvent, elle est de 30 minutes à 8 heures.

Dans une étape suivante du procédé de l'invention, on effectue un traitement d'hydrolyse de la masse réactionnelle obtenue.

La quantité d'eau mise en oeuvre peut varier très largement. Le rapport entre le nombre de moles d'eau et le nombre de moles de composé aromatique peut varier, entre 10 et 100, et de préférence entre 20 et 30.

A cet effet, un mode préféré de réalisation de cette opération consiste à ajouter la masse réactionnelle sur un pied d'eau portée à une température comprise entre 0°C et 100°C, de préférence entre 15°C et 30°C.

Une variante de l'invention consiste à remplacer l'eau par une solution basique, généralement de soude, carbonate ou hydrogénocarbonate de sodium ayant une concentration de 5 à 20 % en poids.

On sépare le catalyseur, de préférence par filtration. Le catalyseur peut être recyclé après séchage.

En fin de réaction, on récupère le produit désiré à savoir la cétone aromatique en phase organique.

On sépare les phases aqueuse et organique.

On lave une ou plusieurs fois, la phase organique, de préférence 2 fois, avec l'eau.

On sépare les phases aqueuse et organique.

On récupère ensuite la cétone aromatique à partir de la phase organique selon les techniques connues, par élimination du solvant organique par distillation ou par cristallisation.

Une autre variante de l'invention consiste à récupérer la cétone aromatique, directement, par distillation de

la phase organique comprenant celle-ci et le catalyseur.

Conformément au procédé de l'invention, on obtient une cétone aromatique qui peut être représentée par la formule (III):

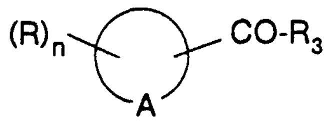

(III)

dans ladite formule (III), A, R, $R_3$ et n ont la signification donnée précédemment.

Un autre objet de la présente invention réside dans la préparation d'un catalyeur répondant à la formule (IV).

Il est préparé avantageusement selon un nouveau procédé qui consiste à faire réagir à basse température, un triarylbismuth et l'acide triflique.

Ainsi, dans la formule (IV), on entend par "Ar", un radical carbocyclique, aromatique, monocyclique ou polycyclique, éventuellement substitué, ayant de préférence, de 6 à 18 atomes de carbone.

On peut citer plus particulièrement, les radicaux phényle ou naphtyle, éventuellement substitués, par exemple, par des radicaux alkyle ou alkoxy ayant de 1 à 4 atomes de carbone, des atomes d'halogène ou des groupes trifluorométhyle. Comme radicaux Ar, on choisit de préférence, un radical phényle ou tolyle et encore plus préférentiellement un radical phényle.

Le triphénylbismuth est le réactif préféré.

La quantité des réactifs mise en oeuvre est généralement telle que le rapport entre le nombre de moles d'acide triflique et le nombre de moles de triarylbismuth est choisi préférentiellement entre 0,9 et 3,1.

Selon la quantité d'acide triflique mis en oeuvre, on obtiendra un catalyseur comprenant ainsi plus ou moins d'anions triflate.

Pour obtenir un catalyseur de formule $Bi(OTf)_3$, la quantité d'acide triflique est de 2,9 à 3,1 moles par mole de triarylbismuth, de préférence égale à 3,0.

Selon une variante de l'invention, on prépare à titre de produits nouveaux, des produits intermédiaires de fabrication du triflate de bismuth qui répondent plus particulièrement à la formule suivante :

$$Ar_n\ Bi(OTf)_{3-n} \qquad (IV)$$

dans laquelle n est égal à 1 ou 2 et Ar et OTf ayant la signification donnée précédemment.

Les composés répondant en particulier aux formules suivantes : $Bi(OTf)Ph_2$ et $Bi(OTf)_2Ph$ sont des produits nouveaux.

Leur préparation dépend de la quantité d'acide triflique mise en oeuvre. Ainsi, pour des catalyseurs de formule (IV) dans laquelle n est égal à 1 et qui est illustrée par la formule (IVb), la quantité d'acide triflique est de 1,9 à 2,1 moles, de préférence 2 moles par mole de triarylbismuth. Dans le cas des catalyseurs de formule (IV) dans laquelle n est égal à 2 et qui est illustrée par la formule (IVa), la quantité d'acide triflique est de 0,9 à 1,1, de préférence 1 mole par mole de triarylbismuth.

La réaction pour préparer le catalyseur est conduite généralement dans un solvant organique.

On fait appel plus particulièrement aux hydrocarbures aliphatiques halogénés, de préférence, au dichlorométhane, chloroforme, tétrachlorure de carbone, 1,2-dichloroéthane.

Le triarylbismuth est présent dans le solvant organique à une concentration comprise de préférence, entre 0,01 et 1 mole/litre.

Selon un mode de mise en oeuvre préféré, l'acide triflique est additionné progressivement dans le milieu comprenant le triarylbismuth et le solvant organique.

En ce qui concerne la température de la réaction, elle est choisie à une temperature inférieure à 0°C, et préférentiellement comprise entre -100°C et -20°C.

En fin de réaction, on laisse le mélange réactionnel revenir à la température ambiante (de préférence, entre 15°C et 25°C) et l'on obtient un triflate de bismuth de formule (IV) sous forme précipitée.

On sépare ledit précipité selon les techniques classiques de séparation solide/liquide, de préférence, par filtration.

Le catalyseur ainsi préparé est mis en oeuvre dans le procédé d'acylation selon l'invention.

L'invention permet d'accéder à de nouveaux catalyseurs, en particulier ceux de formule (IVa) et (IVb). Lesdits

catalyseurs présentent une activité catalytique intéressante et ont l'avantage de ne pas être aussi coûteux que le tris-trifluorométhanesulfonate de bismuth, en raison de la mise en oeuvre d'une quantité moindre d'acide trifluorométhanesulfonique lors de leur préparation.

Les exemples qui suivent, illustrent l'invention sans toutefois la limiter.

Dans les exemples, les rendements mentionnés correspondent à la définition suivante :

$$\text{Rendement} = \frac{\text{nombre de moles de cétone aromatique formées}}{\text{nombre de moles de réactant minoritaire}}\ \%$$

On entend par "réactant minoritaire", soit le substrat aromatique, soit l'agent d'acylation, selon les quantités relatives de chacun introduites.

Exemple 1

Synthèse du triflate de bismuth (III).

Dans un ballon de 250 ml, purgé à l'argon et équipé d'une entrée d'air reliée à un tube à chlorure de calcium, on introduit, sous agitation magnétique, 0,44 g de triphénylbismuth $Ph_3Bi$ (1 mmol) et 10 ml de dichlorométhane anhydre.

La solution est refroidie à - 78°C.

On ajoute, au moyen d'une seringue, 0,44 g d'acide triflique (2,9 mmol).

Une coloration jaune apparaît.

On laisse le mélange réactionnel revenir à température ambiante.

On filtre le solide obtenu sous argon et on le lave avec du dichlorométhane sec afin d'éliminer les traces d'acide triflique.

La poudre est introduite dans un bicol et séchée sous pression réduite (1 mm de mercure), sous argon, pendant 1 heure.

L'analyse de cette poudre par RMN $^{19}F(CD_3COCD_3)$ donne un pic à 0,84 p.p.m., la RMN $^{13}C(CD_3COCD_3)$ donne un quadruplet à $\delta$ = 120 p.p.m. ($^1J_{13C\text{-}19F}$ = 321 Hz).

On note l'absence de signal protonique.

Exemples 2 à 7 :

Acylation de l'anisole en présence de triflate de bismuth.

On définit ci-après le protocole opératoire qui est repris dans les exemples qui suivent.

Dans un ballon de 50 ml, purgé à l'argon, muni d'un réfrigérant équipé d'un tube à dégagement de chlorure de calcium, on introduit le triflate de bismuth (III), le substrat et l'agent acylant.

Les proportions des réactifs sont précisées dans le tableau récapitulatif donné ci-après.

On met en oeuvre 10 mmol de substrat et 5 mmol d'agent acylant pour un rapport molaire de 2.

On introduit le catalyseur à raison de 5 % molaire par rapport au substrat soit 0,16 g.

On ajoute éventuellement 10 ml de solvant.

On porte le mélange à la température réactionnelle (généralement le reflux) mentionnée également dans le tableau récapitulatif.

Après refroidissement, on ajoute 25 ml d'eau.

Les produits organiques sont extraits avec 2x15 ml d'éther éthylique.

La phase organique, séchée sur sulfate de sodium est concentrée et analysée par chromatographie en phase gazeuse.

Les conditions et résultats sont consignés dans le Tableau I.

TABLEAU I

| Réf. ex. | Agent acylant | Rapport molaire anisole/ agent acylant | Solvant | Température | Durée | Rendement (%) |
|---|---|---|---|---|---|---|
| 2 | MeCOCl | 2 | $MeNO_2$ | 50°C | 2 h | 80 |
| 3 | MeCOCl | 1 | sans solvant | 50°C | 3 h | 70 |
| 4 | $Me_2CHCOCl$ | 1 | sans solvant | reflux | 20 mn | 96 |

TABLEAU I (suite)

| Réf. ex. | Agent acylant | Rapport molaire anisole/ agent acylant | Solvant | Température | Durée | Rendement (%) |
|---|---|---|---|---|---|---|
| 5 | PhCOCl | 1 | sans solvant | reflux | 2h 30 mn | 81 |
| 6 | $(MeCO)_2O$ | 2 | $MeNO_2$ | reflux | 4 h | 100 |
| 7 | $(MeCO)_2O$ | 1 | sans solvant | reflux | 1 h | 95 |

Exemples 8 à 10

Acylation d'hydrocarbures aromatiques halogénés ou non en présence de triflate de bismuth.

Dans un ballon de 50 ml, purgé à l'argon, muni d'un réfrigérant équipé d'un tube à dégagement de chlorure de calcium, on introduit 0,16 g (0,5 mmol) de triflate de bismuth (III), 10 mmol de substrat (toluène, benzène, chlorobenzène) et 5 mmol de chlorure de benzoyle.

Le mélange est chauffé sous reflux, pendant la durée indiquée dans le tableau récapitulatif donné ci-après.

Après traitement effectué selon les exemples 2 à 7, on analyse le mélange réactionnel par RMN.

Les conditions et résultats sont rassemblés dans le tableau II.

TABLEAU II

| Réf. ex. | Substrat | Agent acylant | Rapport molaire substrat/ agent acylant | Solvant | Température | Durée | Rendement (%) |
|---|---|---|---|---|---|---|---|
| 8 | Toluène | PhCOCl | 2 | sans solvant | reflux | 8 h | 70 * |
| 9 | Benzène | PhCOCl | 2 | sans solvant | reflux | 4 h | 95 |
| 10 | Chlorobenzène | PhCOCl | 2 | sans solvant | reflux | 4 h | 60 |

* = Proportion d'isomères ortho-para=25/75

Essai comparatif a

On opère comme dans l'exemple 10 à la différence près que l'on remplace le triflate de bismuth par le chlorure de bismuth $BiCl_3$.

Les conditions et résultats sont consignés dans le Tableau III.

TABLEAU III

| Réf. ex. | Substrat | Agent acylant | Rapport molaire substrat/ agent acylant | Solvant | Température | Durée | Rendement (%) |
|---|---|---|---|---|---|---|---|
| a | Chlorobenzène | PhCOCl | 2 | sans solvant | reflux | 4 h | 0 |

Exemples 11 à 16

Dans la série d'exemples qui suit, on effectue la benzoylation de différents substrats qui sont des composés aromatiques porteurs d'un ou de deux substituants X et X' dont la nature est précisée dans le tableau IV.

L'équation réactionnelle est la suivante :

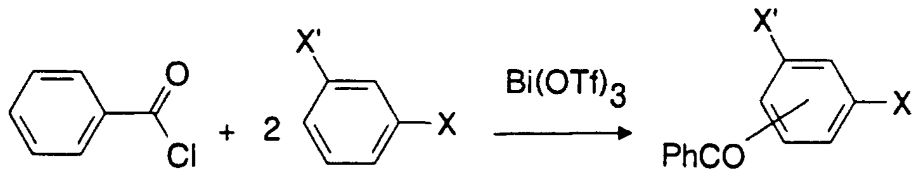


Le protocle opératoire utilisé dans les exemples est donné ci-après :
Dans un ballon de 50 ml, surmonté d'un réfrigérant et équipé d'un tube à dégagement rempli de chlorure de calcium, sont introduits successivement sous argon, $Bi(OTf)_3$ (20 mmol), le substrat (200 mmol) et le chlorure de benzoyle (100 mmol).
Le mélange réactionnel est placé dans un bain d'huile à 140°C : la température dans le ballon est comprise entre 100 et 120°C.
Le mélange est agité magnétiquement pendant 4 heures.
Le brut réactionnel est filtré sur gel de silice (Silicagel Merck 60,230-400 mesh) avec un mélange pentane/éther (1/1) afin d'éliminer le catalyseur.
Les solvants sont évaporés sous pression réduite et le résidu est analysé par chromatographie en phase gazeuse CPG effectuée sur chromatographe Helwett-Packard® GC 6890 (colonne capillaire HP5, 12 m x 0,2 mm, film 5 % phénylméthylsilicone) et par CPG-SM effectuée sur un spectromètre Helwett-Packard® MS 5989 couplé à un chromatographe GC 5890 (colonne capillaire HP1, 12 m x 0,2 mm, film 5 % méthylsilicone, impact électronique).
Les résultats obtenus sont consignés dans le tableau IV.

TABLEAU IV

| Réf. ex. | X | X' | Rendement massique (%) | Sélectivité | | |
|---|---|---|---|---|---|---|
| | | | | o | m | p |
| 11 | $OCH_3$ | H | 74 | 8 | - | 92 |
| 12 | $CH_3$ | H | 95 | 19 | 2 | 79 |
| 13 | H | H | 78 | - | - | - |
| 14 | Cl | H | 89 | 13 | - | 87 |
| 15 | F | H | 86 | - | - | 100 |
| 16 | Cl | Cl | 37 | - | - | - |

<u>Exemples 17 et 18</u>

Dans les deux exemples qui suivent, on prépare deux catalyseurs $Bi(OTf)Ph_2$ et $Bi(OTf)_2Ph$.
Pour ce faire, on répète le mode opératoire de l'exemple 1 à la différence près que l'on introduit 1 mmol d'acide triflique dans l'exemple 17 et 2 mmol d'acide triflique dans l'exemple 18.
L'analyse par RMN [solvant $CD_3COCD_3$ ; δ en ppm, références TMS ($^1H$, $^{13}C$), $CF_3COOH$ ($^{19}F$)].de la poudre $Bi(OTf)Ph_2$ obtenue selon l'exemple 17 est la suivante :

- RMN $^1H$ : on distingue 3 groupes de signaux pour les protons aromatiques : 7,45 ppm (multiplet, 1H, para) 8,28 (multiplet, 2H, méta), 9,28 (multiplet, 2H, ortho) ;
- RMN $^{19}F$ : un signal à 1,18 ppm (singulet) ;
- RMN $^{13}C$ : 120,9 ppm (quadruplet, J = 321 Hz, $CF_3$), 129,7, 133,1, 138,3 (3 carbones CH aromatiques ; le carbone quaternaire n'apparaît pas).

L'analyse par RMN de la poudre $Bi(OTf)_2Ph$ obtenue selon l'exemple 18 est la suivante :

- RMN $^1H$ : on distingue 3 groupes de signaux pour les protons aromatiques : 7,58 ppm (multiplet, 1H, para), 8,28 (multiplet, 2H, méta), 9,28 (multiplet, 2 H, ortho) ;
- RMN$^{19}F$ : un signal à 1,14 ppm (singulet, $CF_3$) ;
- RMN $^{13}C$ : 121,0 ppm (quadruplet, J = 321 Hz, $CF_3$), 130,4, 134,9, 139,1 (3 carbones CH aromatiques ; le carbone quaternaire n'apparaît pas).

Exemples 19, 20 et 21

On reproduit le protocole opératoire des exemples 11 à 16 mais en mettant en oeuvre :

- comme substrat : le toluène,
- comme agent acylant : le chlorure de benzoyle,
- comme catalyseur :
  - . dans l'exemple 19 : le catalyseur de l'exemple 1,
  - . dans les exemples 20 et 21 : les catalyseurs des exemples 18 et 17.

La température réactionnelle est de 120°C et la durée de 4 heures.

Le tableau suivant précise les rendements obtenus :

TABLEAU V

| Ref. ex. | Nature du catalyseur | Rendement massique (%) |
|---|---|---|
| 19 | $Bi(OTf)_3$ | 95 |
| 20 | $Bi(OTf)_2Ph$ | 74 |
| 21 | $Bi(OTf)Ph_2$ | 73 |

On note donc que les produits intermédiaires mis en oeuvre dans les exemples 20 et 21 présentent une activité catalytique intéressante.

## Revendications

1. Procédé d'acylation d'un composé aromatique qui consiste à faire réagir ledit composé aromatique avec un agent d'acylation, en présence d'un catalyseur **caractérisé par le fait que** l'on conduit la réaction d'acylation en présence d'une quantité efficace d'au moins un sel de bismuth de l'acide triflique.

2. Procédé selon la revendication 1 **caractérisé par le fait que** le composé aromatique répond à la formule générale (I) :

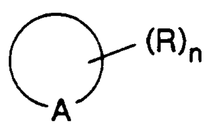

(I)

dans laquelle :

- A symbolise le reste d'un cycle formant tout ou partie d'un système carbocyclique ou hétérocyclique, aromatique, monocyclique ou polycyclique : ledit reste cyclique pouvant porter un radical R représentant un atome d'hydrogène ou un ou plusieurs substituants, identiques ou différents,
- n représente le nombre de substituants sur le cycle.

3. Procédé selon l'une des revendications 1 et 2 **caractérisé par le fait que** le composé aromatique répond à la formule générale (I) dans laquelle le reste A éventuellement substitué représente, le reste :

   1° - d'un composé carbocyclique aromatique, monocyclique ou polycyclique.
   2° - d'un composé hétérocyclique aromatique, monocyclique ou polycyclique.
   3° - d'un composé constitué par un enchaînement de cycles, tels que définis aux paragraphes 1 et/ou 2 liés entre eux :

   - . par un lien valentiel,

- par un radical alkylène ou alkylidène ayant de 1 à 4 atomes de carbone, de préférence un radical méthylène ou isopropylidène,
- par l'un des groupes suivants :

-O-, -CO-, -COO-, -OCOO-

-S-, -SO-, $-SO_2-$,

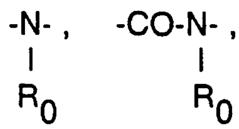

dans ces formules, $R_0$ représente un atome d'hydrogène ou un radical alkyle ayant de 1 à 4 atomes de carbone, un radical cyclohexyle ou phényle.

**4.** Procédé selon l'une des revendications 1 à 3 **caractérisé par le fait que** le composé aromatique répond à la formule générale (I) dans laquelle le ou les radicaux R représentent l'un des groupes suivants :

- un atome d'hydrogène,
- un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
- un radical alcényle linéaire ou ramifié ayant de 2 à 6 atomes de carbone, de préférence, de 2 à 4 atomes de carbone, tel que vinyle, allyle,
- un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
- un radical cyclohexyle,
- un groupe acyle ayant de 2 à 6 atomes de carbone,
- un radical de formule :

$-R_1-OH$

$-R_1-COOR_2$

$-R_1-CHO$

$-R_1-NO_2$

$-R_1-CN$

$-R_1-(NR_2)_2$

$-R_1-CO-(NR_2)_2$

$-R_1-X$

$-R_1-CF_3$

dans lesdites formules, $R_1$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; les radicaux $R_2$ identiques ou différents représentent un atome d'hydrogène ou un radical alkyle linéaire ou ramifié ayant de 1 à 6 atomes de carbone ; X symbolise un atome d'halogène, de préférence un atome de chlore, de brome ou de fluor.

**5.** Procédé selon l'une des revendications 1 à 4 **caractérisé par le fait que** le composé aromatique répond à la formule générale (I) dans laquelle lorsque n est supérieur ou égal à 2, deux radicaux R et les 2 atomes successifs du cycle aromatique peuvent être liés entre eux par un radical alkylène, alcénylène ou alcénylidène ayant de 2 à 4 atomes de carbone pour former un hétérocycle saturé, insaturé ou aromatique ayant de 5 à 7 atomes de carbone : un ou plusieurs atomes de carbone pouvant être remplacés par un autre hétéroatome, de préférence l'oxygène.

**6.** Procédé selon l'une des revendications 1 à 5 **caractérisé par le fait que** le composé aromatique répond à la formule générale (I) dans laquelle :

- le ou les radicaux R représentent l'un des groupes suivants :
  - . un atome d'hydrogène
  - . un groupe OH,
  - . un radical alkyle linéaire ou ramifié ayant de 1 à 6 atomes de carbone,
  - . un radical alcényle linéaire ou ramifié ayant de 2 à 6 atomes de carbone,
  - . un radical alkoxy linéaire ou ramifié ayant de 1 à 6 atomes de carbone,
  - . un groupe -CHO,
  - . un groupe acyle ayant de 2 à 6 atomes de carbone,
  - . un groupe $-COOR_2$ où $R_2$ a la signification donnée précédemment,
  - . un groupe $-NO_2$,
  - . un groupe $-NH_2$,
  - . un atome d'halogène, de préférence, de fluor, de chlore, de brome,
  - . un groupe $-CF_3$.
- n est un nombre égal à 0, 1.2 ou 3.

**7.** Procédé selon l'une des revendications 1 à 6 **caractérisé par le fait que** le composé aromatique répondant à la formule générale (I) est un composé carbocyclique aromatique monocyclique ou polycyclique avec des cycles pouvant former entre eux un système orthocondensé répondant à la formule (Ia) :

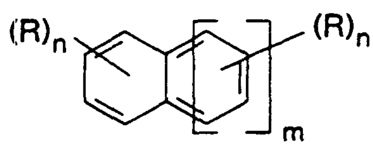

(Ia)

dans ladite formule (Ia), m représente un nombre égal à 0,1 ou 2 et les symboles R, identiques ou différents et n, ayant la signification donnée précédemment.

**8.** Procédé selon l'une des revendications 1 à 6 **caractérisé par le fait que** le composé aromatique répondant à la formule générale (I) est un composé constitué par un enchaînement de deux ou plusieurs carbocycles aromatiques monocycliques répondant à la formule (Ib):

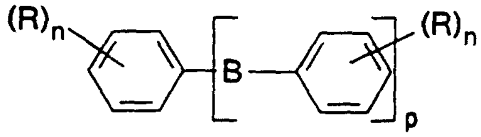

(Ib)

dans ladite formule (Ib), les symboles R, identiques ou différents et n, ont la signification donnée précédemment, p est un nombre égal à 0, 1, 2 ou 3 et B représente :

- un lien valentiel
- un radical alkylène ou alkylidène ayant de 1 à 4 atomes de carbone, de préférence, un radical méthylène ou isopropylidène,
- l'un des groupes suivants :

-O- , -CO-, -COO-, -OCOO-

-S-, -SO-, $-SO_2-$,

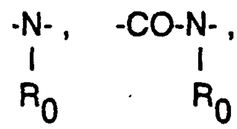

dans ces formules, $R_0$ représente un atome d'hydrogène ou un radical alkyle ayant de 1 à 4 atomes de carbone, un radical cyclohexyle ou phényle.

**9.** Procédé selon l'une des revendications 7 et 8 **caractérisé par le fait que** le composé aromatique répond à la formule (Ia) ou (Ib) dans laquelle :

- R représente un atome d'hydrogène, un groupe hydroxyle, un groupe —CHO, un groupe $-NO_2$, un groupe $-NH_2$, un radical alkyle ou alkoxy linéaire ou ramifié ayant de 1 à 6 atomes de carbone, de préférence, de 1 à 4 atomes de carbone ou un atome d'halogène,
- B symbolise un lien valentiel, un radical alkylène ou alkylidène ayant de 1 à 4 atomes de carbone ou un atome d'oxygène,
- m est égal à 0 ou 1,
- n est égal à 0, 1 ou 2,
- p est égal à 0 ou 1.

**10.** Procédé selon l'une des revendications 1 à 9 **caractérisé par le fait que** le composé aromatique répond à la formule générale (I) dans laquelle R représente un atome d'hydrogène, un groupe hydroxyle, un radical méthyle, un radical méthoxy ou un atome d'halogène.

**11.** Procédé selon la revendication 1 et 2 **caractérisé par le fait que** le composé aromatique est le benzène, le toluène, le phénol, l'anisole ou le vératrole.

**12.** Procédé selon l'une des revendications 1 à 11 **caractérisé par le fait que** l'agent d'acylation répond à la formule (II) :

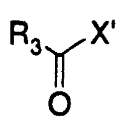

(II)

dans laquelle :

- $R_3$ représente :

  . un radical aliphatique saturé ou insaturé, linéaire ou ramifié ayant de 1 à 24 atomes de carbone ; un radical cycloaliphatique, saturé, insaturé ou aromatique, monocyclique ou polycyclique, ayant de 4 à 12 atomes de carbone ; un radical aliphatique saturé ou insaturé, linéaire ou ramifié, porteur d'un substituant cyclique,

- X' représente :

  . un atome d'halogène, de préférence un atome de chlore ou de brome,
  . un radical -O-CO-$R_4$ avec $R_4$, identique ou différent de $R_3$, ayant la même signification que $R_3$.

**13.** Procédé selon la revendication 12 **caractérisé par le fait que** l'agent d'acylation répond à la formule (II) dans laquelle X' représente un atome de chlore et $R_3$ représente un radical alkyle linéaire ou ramifié ayant de 1 à 12 atomes de carbone : la chaîne hydrocarbonée pouvant être éventuellement interrompue par un hétéroatome ou par un groupe fonctionnel ou porteuse d'un substituant ; $R_3$ représente un radical phényle éventuellement substitué ; ou X' représente un radical -O-CO-$R_3$. dans laquelle $R_3$ et $R_4$ sont identiques et représentent un radical alkyle ayant de 1 à 4 atomes de carbone.

**14.** Procédé selon la revendication 12 et 13 **caractérisé par le fait que** l'agent d'acylation est choisi parmi :

- le chlorure d'acétyle,
- le chlorure de monochloroacétyle,
- le chlorure de dichloroacétyle,
- le chlorure de propanoyle,
- le chlorure d'isobutanoyle,
- le chlorure de pivaloyle,
- le chlorure de stéaroyle,
- le chlorure de crotonyle,
- le chlorure de benzoyle,
- les chlorures de chlorobenzoyle,
- le chlorure de p-nitrobenzoyle
- les chlorures de méthoxybenzoyle,
- les chlorures de naphtoyle,
- l'anhydride acétique,
- l'anhydride isobutyrique,
- l'anhydride trifluoroacétique,
- l'anhydride benzoïque.

**15.** Procédé selon l'une des revendications 1 à 14 **caractérisé par le fait que** le solvant réactionnel est l'un des réactifs ou un solvant organique aprotique, polaire.

**16.** Procédé selon la revendication 15 **caractérisé par le fait que** le solvant organique est choisi parmi les carboxamides linéaires ou cycliques comme le N,N-diméthylacétamide (DMAC), le N,N-diéthylacétamide, le diméthylformamide (DMF), le diéthylformamide ou la 1-méthyl-2-pyrrolidinone (NMP) ; les composés nitrés tels que le nitrométhane, le nitroéthane, le 1-nitropropane, le 2-nitropropane ou leurs mélanges, le nitrobenzène ; les nitriles aliphatiques ou aromatiques comme l'acétonitrile, le propionitrile, le butanenitrile, l'isobutanenitrile, le benzonitrile, le cyanure de benzyle ; le diméthylsulfoxyde (DMSO) ; la tétraméthylsulfone (sulfolane), la diméthylsulfone, l'hexaméthylphosphotriamide (HMPT) ; la diméthyléthylèneurée, la diméthylpropylèneurée, la tétraméthylurée ; le carbonate de propylène ou leurs mélanges.

**17.** Procédé selon l'une des revendications 1 à 16 **caractérisé par le fait que** le rapport entre le nombre de moles de composé aromatique et le nombre de moles d'agent d'acylation varie entre 0,1 et 10, de préférence entre 1,0 et 4,0.

**18.** Procédé selon l'une des revendications 1 à 17 **caractérisé par le fait que** la quantité de catalyseur mis en oeuvre est telle que le rapport entre le nombre de moles de catalyseur et le nombre de moles d'agent d'acylation est

inférieur à 1,0 et varie, de préférence, entre 0,001 et 0,8, et encore plus préférentiellement entre 0,02 et 0,2.

**19.** Procédé selon l'une des revendications 1 à 18 **caractérisé par le fait que** la température à laquelle est mise en oeuvre la réaction d'acylation se situe entre 20°C et 200°C, de préférence entre 40°C et 150°C.

**20.** Procédé selon l'une des revendications 1 à 19 **caractérisé par le fait que** le catalyseur comprend au moins un sel de bismuth de l'acide triflique répondant à la formule (IV) :

$$Ar_n\ Bi(OTf)_{3-n} \qquad (IV)$$

dans ladite formule (IV) :

- OTf représente $O_3SCF_3$,
- Ar représente un radical carbocyclique, aromatique, monocyclique ou polycyclique,
- n est égal à 0, 1 ou 2.

**21.** Procédé selon la revendication 20 **caractérisé par le fait que** le sel de bismuth de l'acide triflique répond à la formule (IV) dans laquelle Ar représente un radical phényle ou naphtyle éventuellement substitué par des radicaux alkyle ou alkoxy ayant de 1 à 4 atomes de carbone, des atomes d'halogène ou des groupes trifluorométhyle.

**22.** Procédé selon l'une des revendications 1 à 21 **caractérisé par le fait que** le catalyseur est choisi parmi $Bi(OTf)_3$, $Bi(OTf)_2Ph$, $Bi(OTf)Ph_2$ et leurs mélanges.

**23.** Sel de bismuth de l'acide triflique répondant à la formule (IV):

$$Ar_n\ Bi(OTf)_{3-n} \qquad (IV)$$

dans ladite formule (IV) :

- OTf représente $O_3SCF_3$,
- Ar représente un radical carbocyclique, aromatique, monocyclique ou polycyclique,
- n est égal à 1 ou 2.

**24.** Sel de bismuth de l'acide triflique selon la revendication 23 **caractérisé par le fait qu'**il répond à la formule (IV) dans laquelle Ar représente un radical phényle ou naphtyle éventuellement substitué par des radicaux alkyle ou alkoxy ayant de 1 à 4 atomes de carbone, des atomes d'halogène ou des groupes trifluorométhyle.

**25.** Sel de bismuth de l'acide triflique de formule $Bi(OTf)_2Ph$ ou $Bi(OTf)Ph_2$.

**26.** Procédé de préparation du tris-trifluorométhanesulfonate de bismuth **caractérisé par le fait qu'**il consiste à faire réagir à une température inférieure à 0°C, un triarylbismuth et l'acide triflique.

**27.** Procédé selon la revendication 26 **caractérisé par le fait que** le rapport entre le nombre de moles d'acide triflique et le nombre de moles de triarylbismuth est choisi entre 2,9 et 3,1, de préférence égal à 3,0.

**28.** Procédé de préparation du sel de bismuth de l'acide triflique répondant à la formule (IV), **caractérisé par le fait qu'**il consiste à faire réagir à une température inférieure à 0°C, un triarylbismuth et l'acide triflique.

**29.** Procédé selon la revendication 28 **caractérisé par le fait que** le rapport entre le nombre de moles d'acide triflique et le nombre de moles de triarylbismuth est choisi : entre 1,9 et 2,1, de préférence égal à 2,0 pour le sel de bismuth de formule (IV) dans laquelle n est égal à 1 ; et entre 0,9 et 1,1, de préférence égal à 1,0 pour le sel de bismuth de formule (IV) dans laquelle n est égal à 2.

**30.** Procédé selon l'une revendications 26 à 29 **caractérisé par le fait que** le triarylbismuth est le triphénylbismuth.

**31.** Procédé selon l'une des revendications 26 à 30 **caractérisé par le fait que** l'on ajoute progressivement l'acide triflique dans le triarylbismuth.

**32.** Procédé selon l'une des revendications 26 à 31 **caractérisé par le fait que** la réaction est conduite généralement dans un solvant organique plus particulièrement un hydrocarbure aliphatique halogéné, de préférence, le dichlorométhane, le chloroforme, le tétrachlorure de carbone et/ou le 1,2-dichloroéthane.

**33.** Procédé selon l'une des revendications 26 à 32 **caractérisé par le fait que** le triarylbismuth est présent dans le solvant organique à une concentration comprise de préférence, entre 0,01 et 1 mole/litre.

**34.** Procédé selon l'une des revendications 26 à 33 **caractérisé par le fait que** la température de la réaction est choisie avantageusement inférieure à 0°C, et plus préférentiellement comprise entre -100°C et -20°C.

**Patentansprüche**

**1.** Verfahren zur Acylierung einer aromatischen Verbindung, bei der diese in Anwesenheit eines Katalysators mit einem Acylierungsmittel umgesetzt wird, **dadurch gekennzeichnet, daß** man die Acylierungsreaktion in Gegenwart einer wirksamen Menge mindestens eines Bismutsalzes der Triflinsäure durchführt.

**2.** Verfahren nach Anspruch 1, **dadurch gekennzeichnet, daß** die aromatische Verbindung der allgemeinen Formel (I) entspricht,

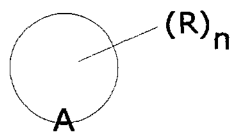

(I)

in der

- A den Rest eines Zyklus symbolisiert, der ganz oder teilweise ein carbocyclisches oder heterocyclisches aromatisches, monocyclisches oder polycyclisches System bildet: wobei der cyclische Rest einen Rest R tragen kann, der ein Wasserstoffatom oder einen oder mehrere gleiche oder verschiedene Substituenten bedeutet,
- n die Anzahl der Substituenten an dem Zyklus bedeutet.

**3.** Verfahren nach einem der Ansprüche 1 und 2, **dadurch gekennzeichnet, daß** die aromatische Verbindung der allgemeinen Formel (I) entspricht, in der der gegebenenfalls substituierte Rest A den Rest darstellt:

1°- einer monocyclischen oder polycyclischen, carbocyclischen aromatischen Verbindung,
2°- einer monocyclischen oder polycyclischen, heterocyclischen aromatischen Verbindung,
3°- einer durch Verknüpfung der in den Absätzen 1 und 2 definierten miteinander verbundenen Zyklen gebildete Verbindung,

- durch eine Valenzbindung,
- durch einen Alkylen- oder Alkylidenrest mit 1 bis 4 Kohlenstoffatomen, vorzugsweise einen Methylen- oder Isopropylidenrest
- durch eine der folgenden Gruppen:

-O-, -CO-, -COO-, -OCOO-

-S-, -SO-, $-SO_2-$,

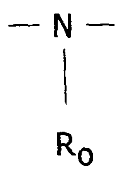 , 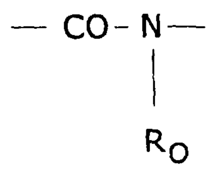 ,

wobei in den Formeln $R_0$ ein Wasserstoffatom oder einen Alkylrest mit 1 bis 4 Kohlenstoffatomen, ein Cyclohexyl- oder Phenylrest bedeutet.

4. Verfahren nach einem der Ansprüche 1 bis 3, **dadurch gekennzeichnet, daß** die aromatische Verbindung der allgemeinen Formel (I) entspricht, in der der oder die Reste R eine der folgenden Gruppen bedeuten:

- ein Wasserstoffatom,
- einen gerad- oder verzweigtkettigen Alkylrest mit 1 bis 6 Kohlenstoffatomen, vorzugsweise 1 bis 4 Kohlenstoffatomen, wie Methyl, Ethyl, Propyl, Isopropyl, Butyl, Isobutyl, sec-Butyl, tert-Butyl,
- einen gerad- oder verzweigtkettigen Alkenylrest mit 2 bis 6 Kohlenstoffatomen, vorzugsweise 2 bis 4 Kohlenstoffatomen, wie Vinyl, Allyl,
- einen gerad- oder verzweigtkettigen Alkoxyrest mit 1 bis 6 Kohlenstoffatomen, vorzugsweise 1 bis 4 Kohlenstoffatomen, wie die Reste Methoxy, Ethoxy, Propoxy, Isopropoxy, Butoxy,
- einen Cyclohexylrest,
- einen Acylrest mit 2 bis 6 Kohlenstoffatomen,
- einen Rest der Formel

$$-R_1-OH$$

$$-R_1-COOR_2$$

$$-R_1-CHO$$

$$-R_1-NO_2$$

$$-R_1-CN$$

$$-R_1-(NR_2)_2$$

$$-R_1-CO-(NR_2)_2$$

$$-R_1-X$$

$$-R_1-CF_3$$

wobei in den genannten Formeln $R_1$ eine Valenzbindung oder einen zweiwertigen, gerad- oder verzweigtkettigen gesättigten oder ungesättigten Kohlenwasserstoffrest mit 1 bis 6 Kohlenstoffatomen, wie z.B. Methylen, Ethylen, Propylen, Isopropylen, Isopropyliden bedeutet; die gleichen oder verschiedenen Reste $R_2$ ein Wasserstoffatom oder einen linearen oder verzweigten Alkylrest mit 1 bis 6 Kohlenstoffatomen bedeuten; X ein Halogenatom, vorzugsweise ein Chlor-, Brom- oder Fluoratom symbolisiert.

**5.** Verfahren nach einem der Ansprüche 1 bis 4, **dadurch gekennzeichnet, daß** die aromatische Verbindung der allgemeinen Formel (I) entspricht, in der n gleich oder größer 2 ist, wobei zwei Reste R und zwei aufeinander folgende Atome des aromatischen Zyklus untereinander durch einen Alkylen-, Alkenylen- oder Alkenylidenrest mit 2 bis 4 Kohlenstoffatomen verbunden sein können, um einen gesättigten, ungesättigten oder aromatischen Heterozyklus mit 5 bis 7 Kohlenstoffatomen zu bilden: wobei einer oder mehrere Kohlenstoffatome durch ein anderes Heteroatom, vorzugsweise Sauerstoff, ersetzt sein können.

**6.** Verfahren nach einem der Ansprüche 2 bis 5, **dadurch gekennzeichnet, daß** die aromatische Verbindung der allgemeinen Formel (I) entspricht, in der der oder die Reste R eine der folgenden Gruppen bedeutet:

- ein Wasserstoffatom,
- eine OH-Gruppe,
- einen gerad- oder verzweigtkettigen Alkylrest mit 1 bis 6 Kohlenstoffatomen,
- einen gerad- oder verzweigtkettigen Alkenylrest mit 2 bis 6 Kohlenstoffatomen,
- einen gerad- oder verzweigtkettigen Alkoxyrest mit 1 bis 6 Kohlenstoffatomen,
- eine CHO-Gruppe,
- eine Acylgruppe mit 2 bis 6 Kohlenstoffatomen,
- eine Gruppierung $-RCOOR_2$, in der R die obige Bedeutung hat,
- eine $-NO_2$-Gruppe,
- eine $-NH_2$-Gruppe,
- ein Halogenatom, vorzugsweise Fluor, Chlor, Brom,
- und eine $-CF_3$-Gruppe
- n gleich 0, 1, 2 oder 3 ist.

**7.** Verfahren nach einem der Ansprüche 1 bis 6, **dadurch gekennzeichnet, daß** die der allgemeinen Formel (I) entsprechende aromatische Verbindung eine monocyclische oder polycyclische, carbocyclische aromatische Verbindung mit Zyklen sein kann, die untereinander ein orthokondensiertes System der Formel (1a) bilden:

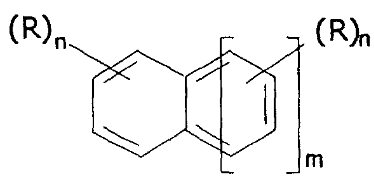

(Ia)

in der m eine ganze Zahl gleich 0, 1 oder 2 ist, und die gleichen oder verschiedenen Symbole R und n die oben gegebene Bedeutung haben.

**8.** Verfahren nach einem der Ansprüche 1 bis 6, **dadurch gekennzeichnet, daß** die der allgemeinen Formel (I) entsprechende aromatische Verbindung eine durch die Verknüpfung von zwei oder mehreren monocyclischen aromatischen Carbocyclen gebildete Verbindung der Formel (Ib) ist:

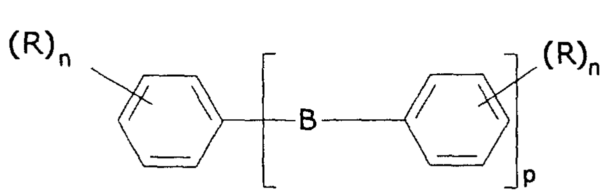

(Ib)

in der die gleichen oder verschiedenen Symbole R und n die zuvor gegebene Bedeutung haben, p eine Zahl von 0, 1, 2 oder 3 ist, und B darstellt:

- eine Valenzbindung,
- einen Alkylen- oder Alkenylidenrest mit 1 bis 4 Kohlenstoffatomen, vorzugsweise einen Methylen- oder Isopropylidenrest,

- eine der folgenden Gruppen:

- O-, -CO-, -COO-, -OCOO-

-S-, -SO-, $-SO_2-$,

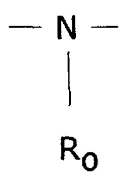 , 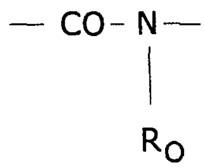 ,

wobei in den Formeln $R_0$ ein Wasserstoffatom oder einen Alkylrest mit 1 bis 4 Kohlenstoffatomen, einen Cyclohexyl- oder Phenylrest bedeutet.

**9.** Verfahren nach einem der Ansprüche 7 oder 8, **dadurch gekennzeichnet, daß** die aromatische Verbindung der Formel (Ia) oder (Ib) genügt, in der:

- R ein Wasserstoffatom, eine Hydroxylgruppe, eine CHO-Gruppe, eine $-NO_2$-Gruppe, eine $-NH_2$-Gruppe, einen gerad- oder verzweigtkettigen Alkyl- oder Alkoxyrest mit 1 bis 6 Kohlenstoffatomen, vorzugsweise 1 bis 4 Kohlenstoffatomen, oder ein Halogenatom bedeutet,
- B eine Valenzbindung, einen Alkylen- oder Alkylidenrest mit 1 bis 4 Kohlenstoffatomen oder ein Sauerstoffatom symbolisiert,
- m gleich 0 oder 1 ist,
- n gleich 0, 1 oder 2 ist,
- p gleich 0 oder 1 ist.

**10.** Verfahren nach einem der Ansprüche 1 bis 9, **dadurch gekennzeichnet, daß** die aromatische Verbindung der Formel (I) entspricht, in der R ein Wasserstoffatom, eine Hydroxylgruppe, einen Methylrest, einen Methoxyrest oder Halogenatom bedeutet.

**11.** Verfahren nach Anspruch 1 oder 2, **dadurch gekennzeichnet, daß** die aromatische Verbindung Benzol, Toluol, Phenol, Anisol oder Veratrol ist.

**12.** Verfahren nach einem der Ansprüche 1 bis 11, **dadurch gekennzeichnet, daß** das Acylierungsmittel der Formel (II) entspricht:

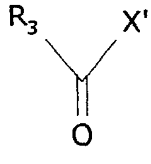

(II)

in der:

- $R_3$ bedeutet:

  - einen gerad- oder verzweigtkettigen, gesättigten oder ungesättigten aliphatischen Rest mit 1 bis 24 Kohlenstoffatomen; einen mono- oder polycyclischen, gesättigten oder ungesättigten oder aromatischen cycloaliphatischen Rest mit 4 bis 12 Kohlenstoffatomen; einen gerad- oder verzweigtkettigen, gesättigten oder ungesättigten aliphatischen Rest mit einem cyclischen Substituenten,

- X' bedeutet:

- ein Halogenatom, vorzugsweise ein Chlor- oder Bromatom,
- einen Rest -O-CO-$R_4$ mit $R_4$, gleich oder verschieden von $R_3$ mit der gleichen Bedeutung wie $R_3$.

**13.** Verfahren nach Anspruch 12, **dadurch gekennzeichnet, daß** das Acylierungsmittel der Formel (II) entspricht, in der X' ein Chloratom und $R_3$ einen linearen oder verzweigten Alkylrest mit 1 bis 12 Kohlenstoffatomen bedeutet: wobei die Kohlenwasserstoffkette gegebenenfalls durch ein Heteroatom oder eine funktionelle Gruppe unterbrochen sein oder einen Substituenten tragen kann; $R_3$ einen gegebenenfalls substituierten Phenylrest bedeutet; wobei X' den Rest -O-CO-$R_3$ darstellt, in dem $R_3$ und $R_4$ identisch sind und einen Alkylrest mit 1 bis 4 Kohlenstoffatomen bedeuten.

**14.** Verfahren nach Anspruch 12 oder 13, **dadurch gekennzeichnet, daß** das Acylierungsmittel ausgewählt ist unter:

- Acetylchlorid,
- Monochloracetylchlorid,
- Dichloracetylchlorid,
- Propanoylchlorid,
- Isobutanoylchlorid,
- Pivaloylchlorid,
- Stearoylchlorid,
- Crotonylchlorid,
- Benzoylchlorid,
- Chlorbenzoylchloriden,
- p-Nitrobenzoylchlorid,
- Methoxybenzoylchloriden,
- Naphtoylchloriden,
- Essigsäureanhydrid,
- Isobuttersäureanhydrid,
- Trifluoressigsäureanhydrid.
- Benzoylchlorid.

**15.** Verfahren nach einem der Ansprüche 1 bis 14, **dadurch gekennzeichnet, daß** das Reaktionslösungsmittel einer der Reagenzien oder ein aprotisches polares organisches Lösungsmittel ist.

**16.** Verfahren nach Anspruch 15, **dadurch gekennzeichnet, daß** das organische Lösungsmittel unter den linearen oder cyclischen Carboxamiden, wie N,N-Dimethylacetamid (DMAC), N,N-Diethylacetamid, Dimethylformamid (DMF), Diethylformamid oder 1-Methyl-2-Pyrrolidinon (NMP); den Nitroverbindungen wie Nitromethan, Nitroethan, 1-Nitropropan, 2-Nitropropan oder deren Gemischen, Nitrobenzol; den aliphatischen oder aromatischen Nitrilen, wie Acetonitril, Propionitril, Butannitril, Isobutannitril, Benzonitril, Benzylcyanid; Dimethylsulfoxid (DMSO); Tetramethylsulfon (Sulfolan), Dimethylsulfon, Hexamethylphosphotriamid (HMPT); Dimethylethylenharnstoff, Dimethylpropylenharnstoff, Tetramethylharnstoff; Propylencarbonat oder deren Gemischen, gewählt ist.

**17.** Verfahren nach einem der Ansprüche 1 bis 16, **dadurch gekennzeichnet, daß** das Verhältnis der Mole der aromatischen Verbindung und der Mole des Acylierungsmittels zwischen 0,1 und 10, vorzugsweise zwischen 1,0 und 4, schwankt.

**18.** Verfahren nach einem der Ansprüche 1 bis 17, **dadurch gekennzeichnet, daß** die Menge des eingesetzten Katalysators so ist, daß das Verhältnis zwischen der Zahl der Mole des Katalysators und der Mole des Acylierungsmittels unter 1 ist und vorzugsweise zwischen 0,001 und 0,8, insbesondere zwischen 0,02 und 0,2 liegt.

**19.** Verfahren nach einem der Ansprüche 1 bis 18, **dadurch gekennzeichnet, daß** die Temperatur, bei der die Acylierungsreaktion durchgeführt wird, zwischen 20 °C und 200 °C, vorzugsweise zwischen 40 °C und 150 °C, liegt.

**20.** Verfahren nach einem der Ansprüche 1 bis 19, **dadurch gekennzeichnet, daß** der Katalysator wenigstens ein Bismutsalz der Triflinsäure der allgemeinen Formel (IV) enthält:

$$Ar_nBi(OTf)_{3-n} \quad \text{(IV)}$$

wobei in der genannten Formel (IV):

- OTf $O_3SCF_3$ bedeutet,
- Ar einen monocyclischen oder polycyclischen, aromatischen carbocyclischen Rest bedeutet,
- n gleich 0, 1 oder 2 ist.

**21.** Verfahren nach Anspruch 20, **dadurch gekennzeichnet, daß** das Bismutsalz der Triflinsäure der Formel (IV) entspricht, in der Ar einen Phenyl- oder Naphtylrest bedeutet, der gegebenenfalls durch Alkyl- oder Alkoxyreste mit 1 bis 4 Kohlenstoffatomen, Halogenatome oder Trifluormethylgruppen substituiert sein kann.

**22.** Verfahren nach einem der Ansprüche 1 bis 21, **dadurch gekennzeichnet, daß** der Katalysator unter $Bi(OTf)_3$, $Bi(OTf)_2Ph$, $Bi(OTf)Ph_2$ oder deren Gemischen gewählt ist.

**23.** Bismutsalz der Triflinsäure entsprechend der Formel (IV):

$$Ar_nBi(OTf)_{3-n} \quad (IV)$$

in der Formel (IV):

- OTf $O_3SCF_3$ bedeutet,
- Ar einen monocyclischen oder polycyclischen, aromatischen carbocyclischen Rest bedeutet,
- n gleich 1 oder 2 ist.

**24.** Bismutsalz der Triflinsäure nach Anspruch 23, **dadurch gekennzeichnet, daß** es der Formel (IV) entspricht, in der Ar einen Phenyl- oder Naphthylrest bedeutet, der gegebenenfalls durch Alkyl- oder Alkoxyreste mit 1 bis 4 Kohlenstoffatomen, Halogenatome oder Trifluormethylgruppen substituiert ist.

**25.** Bismutsalz der Triflinsäure der Formel $Bi(OTf)_2Ph$ oder $Bi(OTf)Ph_2$.

**26.** Verfahren zur Herstellung von Bismut-tris-trifluormethansulfonat, **dadurch gekennzeichnet, daß** man bei einer Temperatur unter 0 °C ein Triarylbismut und Triflinsäure miteinander reagieren läßt.

**27.** Verfahren nach Anspruch 26, **dadurch gekennzeichnet, daß** das Verhältnis zwischen der Anzahl der Mole der Triflinsäure und der Anzahl der Mole des Triarylbismut zwischen 2,9 und 3,1, vorzugsweise gleich 3,0, gewählt wird.

**28.** Verfahren zur Herstellung eines Bismutsalzes der Triflinsäure entsprechend der Formel (IV), **dadurch gekennzeichnet, daß** man bei einer Temperatur unter 0 °C ein Triarylbismut und Triflinsäure miteinander reagieren läßt.

**29.** Verfahren nach Anspruch 28, **dadurch gekennzeichnet, daß** das Verhältnis der Molzahl der Triflinsäure und der Molzahl des Triarylbismut jeweils gewählt ist: zwischen 1,9 und 2,1, vorzugsweise 2,0, für das Bismutsalz der Formel (IV), in der n gleich 1 ist; und zwischen 0,9 und 1,1, vorzugsweise gleich 1, für das Bismutsalz der Formel (IV), in der n gleich 2 ist.

**30.** Verfahren nach einem der Ansprüche 26 bis 29, **gekennzeichnet durch** die Tatsache, daß das Triarylbismut Triphenylbismut ist.

**31.** Verfahren nach einem der Ansprüche 26 bis 30, **dadurch gekennzeichnet, daß** man die Triflinsäure nach und nach dem Triarylbismut zusetzt.

**32.** Verfahren nach einem der Ansprüche 26 bis 31, **dadurch gekennzeichnet, daß** die Reaktion im allgemeinen in einem organischen Lösungsmittel, insbesondere einem halogenierten aliphatischen Kohlenwasserstoff, vorzugsweise Dichlormethan, Chloroform, Tetrachlorkohlenstoff und/oder 1,2-Dichlorethan, durchgeführt wird.

**33.** Verfahren nach einem der Ansprüche 26 bis 32, **dadurch gekennzeichnet, daß** das Triarylbismut in dem organischen Lösungsmittel in einer Konzentration von vorzugsweise 0,01 bis 1 mol/l vorliegt.

**34.** Verfahren nach einem der Ansprüche 26 bis 33, **dadurch gekennzeichnet, daß** die Reaktionstemperatur vorzugsweise unter 0 °C, insbesondere zwischen -100 °C und -20 °C, gewählt ist.

## Claims

**1.** A process for acylating an aromatic compound, consisting of reacting said aromatic compound with an acylation agent in the presence of a catalyst, **characterized in that** the acylation reaction is carried out in the presence of an effective quantity of at least one bismuth salt of triflic acid.

**2.** A process according to claim 1, **characterized in that** the aromatic compound has general formula (I):

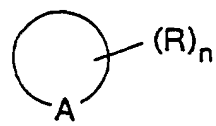

(I)

in which:

- A represents the residue of a cycle forming all or a portion of a monocyclic or polycyclic, aromatic carbocyclic or heterocyclic system: said cyclic residue may carry a radical R representing a hydrogen atom or one or more substituents that may be identical or different;
- n is the number of substituents on the cycle.

**3.** A process according to claim 1 or claim 2, **characterized in that** the aromatic compound has general formula (I) in which residue A, which may be substituted, represents:

1° - a residue of a monocyclic or polycyclic, aromatic carbocylic compound;
2° - a residue of a monocyclic or polycyclic, aromatic heterocyclic compound;
3° - a residue of a compound constituted by a concatenation of cycles as defined in paragraphs 1 and/or 2 bonded together:

- by a covalent bond;
- by an alkylene or alkylidene radical containing 1 to 4 carbon atoms, preferably a methylene or isopropylidene radical;
- by one of the following groups:

-O-, -CO-, -COO-, -OCOO-,

-S-, -SO-, $-SO_2-$,

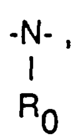 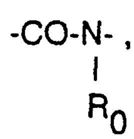

in which $R_0$ represents a hydrogen atom or an alkyl radical containing 1 to 4 carbon atoms, a cyclohexyl radical or a phenyl radical.

**4.** A process according to any one of claims 1 to 3, **characterized in that** the aromatic compound has general formula (I) where radical or radicals R represent one of the following groups:

- a hydrogen atom;
- a linear or branched alkyl radical containing 1 to 6 carbon atoms, preferably 1 to 4 carbon atoms, such as methyl, ethyl, propyl, isopropyl, butyl, isobutyl, sec-butyl or tert-butyl;
- a linear or branched alkenyl radical containing 2 to 6 carbon atoms, preferably 2 to 4 carbon atoms, such as vinyl or allyl;
- a linear or branched alkoxy radical containing 1 to 6 carbon atoms, preferably 1 to 4 carbon atoms, such as methoxy, ethoxy, propoxy, isopropoxy or butoxy;
- a cyclohexyl radical;
- an acyl group containing 2 to 6 carbon atoms;
- a radical with formula:
    - $-R_1-OH$
    - $-R_1-COOR_2$
    - $-R_1-CHO$
    - $-R_1-NO_2$
    - $-R_1-CN$
    - $-R_1-(NR_2)_2$
    - $-R_1-CO-(NR_2)_2$
    - $-R_1-X$
    - $-R_1-CF_3$

in which $R_1$ represents a covalent bond or a saturated or unsaturated, linear or branched divalent hydrocarbon radical containing 1 to 6 carbon atoms, such as methylene, ethylene, propylene, isopropylene or isopropylidene; radicals $R_2$, which may be identical or different, represent a hydrogen atom or a linear or branched alkyl radical containing 1 to 6 carbon atoms; and X represents a halogen atom, preferably a chlorine, bromine or fluorine atom.

**5.** A process according to any one of claims 1 to 4, **characterized in that** the aromatic compound has general formula (I) in which when n is 2 or more, two radicals R and the 2 successive atoms on the aromatic ring can be bonded together via an alkylene, alkenylene or alkenylidene radical containing 2 to 4 compound atoms to form a saturated, unsaturated or aromatic heterocycle containing 5 to 7 carbon atoms: one or more carbon atoms may be replaced by a further heteroatom, preferably oxygen.

**6.** A process according to any one of claims 1 to 5, **characterized in that** the aromatic compound has general formula (I) in which:

- radical or radicals R represent one of the following groups:
    - a hydrogen atom;
    - an OH group;
    - a linear or branched alkyl radical containing 1 to 6 carbon atoms;
    - a linear or branched alkenyl radical containing 2 to 6 carbon atoms;
    - a linear or branched alkoxy radical containing 1 to 6 carbon atoms;
    - a -CHO group;
    - an acyl group containing 2 to 6 carbon atoms;
    - a $-COOR_2$ group where $R_2$ has the meaning given above;
    - a $-NO_2$ group;
    - a $-NH_2$ group;
    - a halogen atom, preferably fluorine, chlorine or bromine;
    - a $-CF_3$ group;
- n is a number equal to 0, 1, 2 or 3.

**7.** A process according to any one of claims 1 to 6, **characterized in that** the aromatic compound with general formula (I) is a monocyclic or polycyclic aromatic carbocyclic compound the cycles of which can between them form an orthocondensed system with formula (Ia):

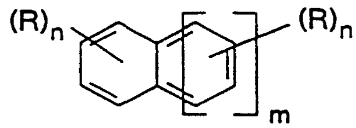

(Ia)

in which formula (Ia), m represents a number equal to 0, 1 or 2, and symbols R, which may be identical or different, and n have the meanings given above.

**8.** A process according to any one of claims 1 to 6, **characterized in that** the aromatic compound with general formula (I) is a compound constituted by a concatenation of two of more monocyclic aromatic carbocycles with formula (Ib):

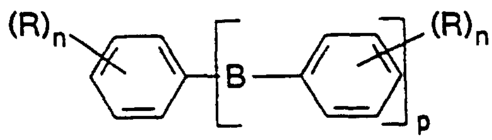

(Ib)

in which formula (Ib), symbols R, which may be identical or different, and n have the meanings given above, p is a number equal to 0, 1, 2 or 3 and B represents:

- a covalent bond;
- an alkylene or alkylidene radical containing 1 to 4 carbon atoms, preferably a methylene or isopropylidene radical;
- one of the following groups:

-O-, -CO-, -COO-, -OCOO-,

- S-, -SO-, $-SO_2-$,

25/20

in which $R_0$ represents a hydrogen atom or an alkyl radical containing 1 to 4 carbon atoms, a cyclohexyl radical or a phenyl radical.

**9.** A process according to claim 7 or claim 8, **characterized in that** the aromatic compound has formula (Ia) or (Ib) in which:

- R represents a hydrogen atom, a hydroxyl group, a -CHO group, a $-NO_2$ group, a $-NH_2$ group, a linear or branched alkyl or alkoxy group containing 1 to 6 carbon atoms, preferably 1 to 4 carbon atoms, or a halogen atom;
- B symbolises a covalent bond, an alkylene or alkylidene radical containing 1 to 4 carbon atoms or an oxygen atom;
- m is equal to 0 or 1;
- n is equal to 0, 1 or 2;
- p is equal to 0 or 1.

**10.** A process according to any one of claims 1 to 9, **characterized in that** the aromatic compound has general formula (I) in which R represents a hydrogen atom, a hydroxyl group, a methyl radical, a methoxy radical or a halogen atom.

**11.** A process according to claim 1 or claim 2, **characterized in that** the aromatic compound is benzene, toluene, phenol, anisole or veratrole.

**12.** A process according to any one of claims 1 to 11, **characterized in that** the acylation agent has formula (II):

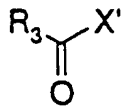

(II)

in which:

- $R_3$ represents:
  - a linear or branched, saturated or unsaturated aliphatic radical containing 1 to 24 carbon atoms; a monocyclic or polycyclic, saturated, unsaturated or aromatic cycloaliphatic radical containing 4 to 12 carbon atoms; or a linear or branched, saturated or unsaturated aliphatic radical carrying a cyclic substituent;
- X' represents:
  - a halogen atom, preferably a chlorine or bromine atom;
  - a -O-CO-$R_4$ radical where $R_4$, which may be identical to or different from $R_3$, has the same meaning as $R_3$.

**13.** A process according to claim 12, **characterized in that** the acylation agent has formula (II) in which X' represents a chlorine atom and $R_3$ represents a linear or branched alkyl radical containing 1 to 12 carbon atoms: the hydrocarbon chain can optionally be interrupted by a heteroatom or by a functional group, or it may carry a substitutent; $R_3$ represents a phenyl radical that may be substituted; or X' represents a -O-CO-$R_3$ radical, where $R_3$ and $R_4$ are identical and represent an alkyl radical containing 1 to 4 carbon atoms.

**14.** A process according to claim 12 or claim 13, **characterized in that** the acylation agent is selected from:

- acetyl chloride;
- monochloroacetyl chloride;
- dichloroacetyl chloride;
- propanoyl chloride;
- isobutanoyl chloride;
- pivaloyl chloride;
- stearoyl chloride;
- crotonyl chloride;
- benzoyl chloride;
- chlorobenzoyl chlorides;
- p-nitrobenzoyl chloride;
- methoxybenzoyl chlorides;
- naphthoyl chlorides;
- acetic anhydride;
- isobutyric anhydride;
- trifluoroacetic anhydride;
- benzoic anhydride.

**15.** A process according to any one of claims 1 to 14, **characterized in that** the reaction solvent is one of the reactants or a polar aprotic solvent.

**16.** A process according to claim 15, **characterized in that** the organic solvent is selected from linear or cyclic carboxamides such as N,N-dimethylacetamide (DMAC), N,N-diethylacetamide, dimethylformamide (DMF), diethylformamide or 1-methyl-2-pyrrolidinone (NMP); nitro compounds such as nitromethane, nitroethane, 1-nitropropane, 2-nitropropane and mixtures thereof, or nitrobenzene; aliphatic or aromatic nitriles such as acetonitrile, propionitrile, butanenitrile, isobutanenitrile, benzonitrile or benzyl cyanide; dimethylsulphoxide (DMSO); tetramethylsulphone (sulpholane), dimethylsulphone, hexamethylphosphotriamide (HMPT); dimethylethylene urea, dimethylpropylene urea and tetramethyl urea; and propylene carbonate or mixtures thereof.

**17.** A process according to any one of claims 1 to 16, **characterized in that** the ratio between the number of moles of aromatic compound and the number of moles of acylation agent is in the range 0.1 to 10, preferably in the range 1.0 to 4.0.

**18.** A process according to any one of claims 1 to 17, **characterized in that** the quantity of catalyst employed is such that the ratio between the number of moles of catalyst and the number of moles of acylation agent is less than 1.0 and preferably in the range 0.001 to 0.8, more preferably in the range 0.02 to 0.2.

**19.** A process according to any one of claims 1 to 18, **characterized in that** the temperature at which the acylation reaction is carried out is in the range 20°C to 200°C, preferably in the range 40°C to 150°C.

**20.** A process according to any one of claims 1 to 19, **characterized in that** the catalyst comprises at least one bismuth salt of triflic acid having formula (IV):

$$Ar_nBi(OTf)_{3-n} \quad (IV)$$

in which formula (IV):

- OTf represents $O_3SCF_3$;
- Ar represents a monocyclic or polycyclic, aromatic carbocylic radical;
- n equals 0, 1 or 2.

**21.** A process according to claim 20, **characterized in that** the bismuth salt of triflic acid has formula (IV) where Ar represents a phenyl or naphthyl radical that may be substituted by alkyl or alkoxy radicals containing 1 to 4 carbon atoms, halogen atoms or trifluoromethyl groups.

**22.** A process according to any one of claims 1 to 21, **characterized in that** the catalyst is selected from $Bi(OTf)_3$, $Bi(OTf)_2Ph$, $Bi(OTf)Ph_2$ and mixtures thereof.

**23.** A bismuth salt of triflic acid having formula (IV):

$$Ar_nBi(OTf)_{3-n} \quad (IV)$$

in which formula (IV):

- OTf represents $O_3SCF_3$;
- Ar represents a monocyclic or polycyclic, aromatic carbocylic radical;
- n equals 0, 1 or 2.

**24.** A bismuth salt of triflic acid according to claim 23, **characterized in that** it has formula (IV) where Ar represents a phenyl or naphthyl radical that may be substituted by alkyl or alkoxy radicals containing 1 to 4 carbon atoms, halogen atoms or trifluoromethyl groups.

**25.** A bismuth salt of triflic acid with formula $Bi(OTf)_2Ph$ or $Bi(OTf)Ph_2$.

**26.** A process for preparing bismuth tris-trifluoromethanesulphonate, **characterized in that** it consists of reacting a triarylbismuth and triflic acid at a temperature of less than 0°C.

**27.** A process according to claim 26, **characterized in that** the ratio between the number of moles of triflic acid and the number of moles of triarylbismuth is in the range 2.9 to 3.1, preferably equal to 3.0.

**28.** A process for preparing bismuth salts of triflic acid with formula (IV), **characterized in that** it consists of reacting a triarylbismuth and triflic acid at a temperature of less than 0°C.

**29.** A process according to claim 28, **characterized in that** the ratio between the number of moles of triflic acid and

the number of moles of triarylbismuth is selected from: in the range 1.9 to 2.1, preferably 2.0 for the bismuth salt with formula (IV) where n equals 1; and in the range 0.9 to 1.1, preferably 1.0 for the bismuth salt with formula (IV) where n equals 2.

**30.** A process according to any one of claims 26 to 29, **characterized in that** the triarylbismuth is triphenylbismuth.

**31.** A process according to any one of claims 26 to 30, **characterized in that** the triflic acid is gradually added to the triarylbismuth.

**32.** A process according to any one of claims 26 to 31, **characterized in that** the reaction is generally carried out in an organic solvent, more particularly a halogenated aliphatic hydrocarbon, preferably dichloromethane, chloroform, carbon tetrachloride and/or 1,2-dichloroethane.

**33.** A process according to any one of claims 26 to 32, **characterized in that** the triarylbismuth is present in the organic solvent in a concentration that is preferably in the range 0.01 to 1 mole/litre.

**34.** A process according to any one of claims 26 to 33, **characterized in that** the reaction temperature is advantageously less than 0°C, more preferably in the range -100°C to -20°C.